# EUROPEAN PATENT SPECIFICATION

(11) **EP 3 426 177 B1**
(45) Date of publication and mention of the grant of the patent: **29.01.2025**
(21) Application number: 17771093.6
(22) Date of filing: 22.03.2017
(51) Int. Cl.: A61B 18/14, A61B 18/00, A61B 18/18

(54) **DEVICES FOR ENERGY DELIVERY AND THERAPY**
VORRICHTUNGEN ZUR ENERGIEABGABE UND THERAPIE
DISPOSITIFS DE DISTRIBUTION D'ÉNERGIE ET DE THÉRAPIE

(30) Priority: 22.03.2016 US 201662311839 P
(43) Date of publication of application: 16.01.2019
(60) Divisional application: 24218342.4
(73) Proprietor: Microcube, LLC, Fremont, CA 94539 (US)
(72) Inventor: WALKE, Amrish J., Milpitas, California 95035 (US); MODY, Dinesh I., San Jose, California 95127 (US); SHROFF, Ketan, Pleasanton, California 94588 (US); TATE, Christine M., Sunnyvale, California 94085 (US)
(74) Representative: Thoma, Michael
(86) International application number: PCT/US2017/023660
(87) International publication number: WO 2017/165562

(56) References cited:
- US-A- 5 364 392
- US-A1- 2010 121 319
- US-A1- 2010 168 727
- US-A1- 2013 150 845
- US-A1- 2013 256 302
- US-A1- 2014 358 140
- US-A9- 2014 190 960

## Description

### BACKGROUND

Several medical methods use energy delivery to perform a variety of actions. During energy delivery, the characteristics of target tissue change which in turn affects the energy delivery. Tissue changes and other phenomenon such as vapor or steam generation may alter the energy delivery during a medical procedure (e.g. by increasing returned power, increasing impedance, increasing temperature, reducing tissue water content, etc.) making it one or more of: less effective, unsafe, too long, too painful, and unpredictable. This is especially important in procedures where the patient is awake. A procedure that takes too long can increase the discomfort experienced by the patient. An exemplary system according to the preamble of claim 1 is disclosed in US 2010/168727.

Thus, there is a need for devices and methods that will account and/or adjust for tissue changes and other phenomenon, including but not limited to vapor or steam generation during an energy delivery procedure. Such devices and methods can deliver an accurate energy dose to increase the effectiveness of the procedure as well as increase safety, speed and/or reduce pain to the patient while still producing predictable outcomes.

### SUMMARY

The invention provides a system according to claim 1. Embodiments of the invention are defined in the dependent claims. For better understanding of the invention , the present disclosure includes also further exemplary methods, said methods not being claimed and not forming part of the invention, and systems
for calculating an energy dose to be delivered to the target material.

### BRIEF DESCRIPTION OF THE FIGURES

Fig. 1 shows a diagram of a microwave energy path of a microwave delivery system for use as described herein.
Figs. 2A - 2C show example tables of modular adjustment of one or more Jt parameters.
Fig. 3 shows an example of a process used in the present disclosure for delivering a Jt dose of microwave energy to tissue.
Fig. 4 shows graphs of reflection coefficients (S-parameter S.sub.11) of an antenna at three different time periods during an ablation procedure.
Fig. 5A shows a comparison of two return power (RP) curves with and without using the methods disclosed herein.
Fig. 5B shows a graph of RP relative to time using a system of the present invention comprising four RP limits.
Fig. 6A shows a graph of Jt versus time for an energy delivery procedure comprising multiple pauses.
Fig. 6B shows graphs of RP and power versus time for an energy delivery procedure comprising multiple changes in energy delivery parameters.
Figs. 7A and 7B an illustration comprising venting one or more products generated because of energy delivery.
Figs. 8A and 8B illustrates venting energy delivery products from the anatomy by moving one or more device regions.
Figs. 9A and 9B show an embodiment of venting energy delivery products from the anatomy by altering the position of a hot spot of all antenna.
Figs. 10A - 10C show the steps of a method of creating or opening a vent for venting energy delivery products from the anatomy.
Figs. 11A - 11I shows views of a display screen during one embodiment of a microwave energy delivery procedure.

### DETAILED DESCRIPTION

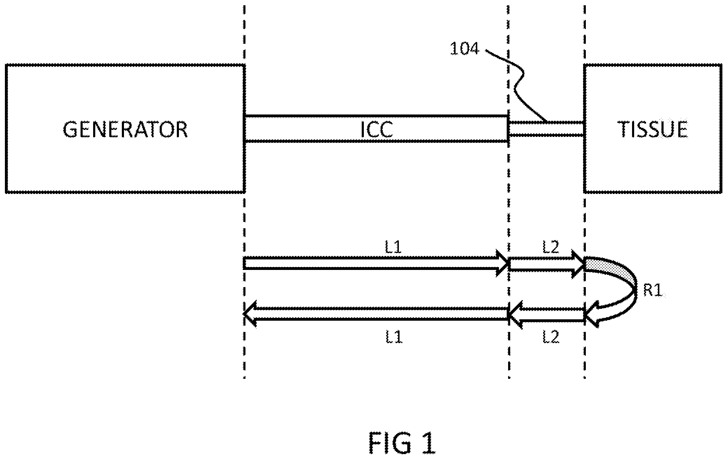

Fig. 1 shows a diagram of a microwave energy path of an example f a microwave delivery system in a variation of the invention described herein. Microwave energy is generated by a Generator and delivered through an interconnecting cable (ICC) to a device comprising an antenna 104. Antenna 104 in turn delivers the microwave energy to Tissue. For simplification of the analysis, a transmission line portion of the device is combined with the ICC as shown in Fig. 1. Fig. 1 also shows the losses and reflections through the microwave energy path. Microwave energy (forward power) transmitting through the ICC experiences a loss L1. Thereafter, microwave energy (forward power) transmitting through the antenna 104 experiences a loss L2. A portion of the microwave energy (forward power) received by the antenna is reflected from the antenna/tissue interface (shown as R1) and a portion is delivered to tissue. R1 represents microwave energy (returned power) coming back from the antenna 104. R1 also experiences losses L2 and L1 on its path back to the generator. For the sake of simplicity, losses and reflections at other interfaces are omitted.

During an energy delivery procedure, R1 is expected to change with time. Two important factors affecting R1 are tissue impedance changes (e.g. due to tissue desiccation, charring, etc.) and accumulation of vapor within or around the tissue. Vapor as mentioned in this specification includes any gaseous products generated because of energy delivery. Examples of such products include, but are not limited to: gaseous state of water formed at temperatures higher or lower than the boiling point of water or tissue fluids.

Although a majority of the embodiments described herein related to microwave or radiofrequency energy delivery, the invention can also be applied to other methods and devices of energy delivery. Examples of such other methods and devices of energy delivery include, but are not limited to: methods and devices for delivering: high voltage, high charge, ultrasound, capacitive coupling, cryotherapy, resistive heating, laser, ionizing radiation, and elements for introducing one or more fluids.

Returned power (RP) measurements include, but are not limited to measurements of returned power magnitude and/or phase, ratio of the returned power to the incident power, characteristics of a standing wave on a transmission line (including, but not limited to: standing wave ratio, voltage standing wave ratio, power standing wave ratio), mismatch between the tissue impedance and the antenna impedance, reflection coefficient, return loss, and the S-parameter S.sub.11.

In one embodiment, RP is measured at the antenna level as a fraction of the power reflected back to the antenna from tissue to the total forward power from the antenna to the tissue.

The system may comprise one or more fixed or user programmable RP limits (RPL) that are used to take one or more decisions during energy delivery. Examples of such limits and decisions are disclosed in US patent US9462642 and related patent filings listed above. RPL may be used for one or more of: detecting component disconnection(s), detecting or stopping inefficient energy delivery, preventing over heating of a transmission line, etc. In one embodiment, an energy delivery system comprises at least two RPLs. A first RPL is used for detecting or stopping inefficient energy delivery and a second, higher RPL is used for detecting component disconnection(s). In one such embodiment, the reaction time of the system to the RP reaching the RPL may be significantly shorter (e.g. < 1 s) for the second RPL and significantly longer (e.g. a few seconds) for the first RPL.

In any of the embodiments herein, generation and/or accumulation of vapor within or around tissue can be detected and/or measured by one or more of:
1. Measuring or analyzing one or more RP parameters such as: one or more changes in RP relative to time, rate of rise of RP with time, one or more RP measurements, inflection point(s) in the curve of RP with another parameter such as time, first or higher derivatives of RP curve against another parameter such as time, impact of one or more trigger(s) on RP, and fluctuations in RP against another parameter such as time. Triggers are defined as any actions that affect vapor around an antenna 104 including actions that change the relative orientation of the vapor and antenna 104. Examples of such triggers include, but are not limited to: actions that increase vapor around antenna 104 (e.g. increasing a power level, increasing a treatment temperature), actions that reduce vapor around antenna 104 (e.g. decreasing a power level, decreasing a treatment temperature, introduction of a cooling fluid) and motion of antenna 104,
2. Visually detecting vapor or other gases coming out of a device or tissue,
3. Measuring or detecting pain experienced by the patient,
4. Measuring temperature(s) at one or more regions of a device or tissue,
5. Detecting tissue movement,
6. Measuring or estimating pressure(s) at one or more regions of a device or tissue,
7. Using an imaging modality (e.g. ultrasound, MRI, X-rays, fluoroscopy, etc.)
8. Using a surrogate marker to measure temperature at one or more regions of a device or tissue. Examples of such markers include, but are not limited to thermochromic materials present on one or more device and/or tissue regions.

In any of the embodiments herein, detection and/or measurement of generation and/or accumulation of vapor within or around tissue can be used for one or more of:
1. Performing one or more steps of a diagnostic and/or therapeutic procedure in response,
2. Detecting perforation of one or more tissue regions e.g. anatomical cavities. In one such embodiment, steam entrapment in a cavity is detected by an increase in RP and is used to conclude that the cavity is not perforated,
3. Detecting abnormal placement and/or deployment of a device, and
4. Monitoring temperature of one or more tissue and/or device regions.

In any of the embodiments herein, one or more energy delivery methods and combinations thereof may be used for one or more of:
A. prevent and/or reduce the accumulation of vapor within or around tissue.
B. prevent and/or reduce the formation or steam or charring.
C. increase the efficiency of energy delivery by an antenna or other energy delivery element. In one such embodiment, a greater percentage of energy is delivered by an antenna to tissue for the same output of a generator supplying energy to the antenna.

Examples of such methods include, but are not limited to one or more of:
1. Venting vapor through one or more vents in a device or in tissue. The venting may be assisted by a vacuum connected to a device or tissue. In one embodiment, a device inserted into the anatomy comprises one or more vents. In one embodiment, a device comprising one or more vents or vent lumens is introduced into the anatomy such that a vent lumen extends from a tissue region where the vapor is being generated and vents the vapor to the atmosphere. The lumens may be collapsible or rigid. The vents or lumens may be present within one or more of: an energy delivery device, a sheath or the space between an energy delivery device and a sheath. In one embodiment, a sheath of sufficient dimensions is provided such that the gap between the sheath and a device acts as a vent. In one embodiment, venting is performed by one or more natural or artificial vents in tissue,
2. Moving one or more of an energy delivery device, a sheath or other device covering the energy delivery device, an antenna, tissue or portions thereof,
3. Introducing/injecting water or other fluids, other non-fluid materials, etc. into tissue or around antenna 104,
4. Introducing a venting device into or adjacent to a tissue "hot spot" or region of localized high temperature. The venting device may be translated and/or rotated within tissue. In one embodiment, the venting device is used to create a vent between a hot spot and the atmosphere. Examples of venting devices include, but are not limited to: devices comprising one or more loops, devices comprising one or more lumens, and devices comprising one or more curved or bent regions, etc.
5. Providing one or more hydrophobic or hydrophilic materials or surface,
6. Compressing one or more tissue regions. In one embodiment, tissue contractions are induced by drugs. In another embodiment, tissue is mechanically compressed by a human or by a device,
7. Expanding or otherwise enlarging one or more tissue regions e.g. using pressure,
8. Changing a dimension of one or more device portions and/or changing a shape of one or more device portions. Examples of such methods include, but are not limited to: inflating a balloon, pulling or pushing one or more device regions, and translating and/or rotating one or more device regions,
9. Introducing colder condensing devices or substances and
10. Altering energy delivery parameters. Any energy delivery parameter including, but not limited to power level, energy delivery duration, size/shape of an energy delivery pulse, duty cycle, energy dose, deployed configuration (including size and shape) of a working element, location of a working element, treatment temperature, and treatment target may be altered. The energy delivery may be continuous or non-continuous. In one embodiment, energy delivery is paused (power level = zero) for some time to allow vapor to condense. Thereafter, energy delivery is restarted. During the pause, the condensation may cause the RP to reduce such that the subsequent energy delivery is more efficient when started. If energy delivery is restarted after a pause, the target energy dose (Jt) may be adjusted to account for the cooling of tissue by blood flow during the pause. Jt adjustment may be calculated based on one or more parameters including, but not limited to: duration of the pause, perfusion rate through a tissue, tissue temperature, etc.

In any of the embodiments herein, one or more methods and combinations thereof may be used to prevent and/or reduce the formation of vapor or charring within or around tissue. Examples of such methods include, but are not limited to one or more of:
1. Altering one or more treatment parameters.

Examples of treatment parameters include, but are not limited to: power level, energy delivery duration, size/shape/other characteristics of an energy delivery pulse, duty cycle, energy dose, deployed configuration (including size and shape) of a working element, duration and presence of one or more pauses during energy delivery, continuous or non-continuous energy delivery, locations of a hot spot relative to a tissue or a device, treatment temperature, and treatment target Locations of a hot spot relative to a tissue or a device may be changed by one or more of: mechanically moving one or more regions of a tissue or a device, altering the characteristics (e.g. frequency) of energy delivery, and changing the dielectric properties of regions surrounding the hot spot. Dielectric properties of regions surrounding the hot spot may be changed by changing the matching of the antenna relative to the surroundings by using one or more of: fluids, mechanical spacers, balloons, and introduction/removal of one or more dielectric materials. The location of a hot spot relative to a tissue or a device may be changed temporarily or multiple times during a procedure.

### 2. Altering antenna design

The following changes may be made to antennas disclosed in any of the following patent applications: US 2014/0190960, US 2010/0137857, US 2010/0121319.

Any of the antennas disclosed in the referenced patent documents may be altered to reduce one or more hot spots. Examples of methods to reduce hot spots include, but are not limited to: adding extra layers of dielectrics or having a thicker dielectric over hot spots, asymmetric antenna designs based on hot spot location(s), and using heat resistant dielectrics, properties of which don't change under typical operating conditions. In one embodiment, one or more dielectrics melt or otherwise get removed during use. In one embodiment, the dielectric effect of one or more dielectrics increases during use. Examples of such dielectrics include, but are not limited to: dielectrics that foam during use, dielectrics that swell during use, etc.

Any of the antennas disclosed in the referenced patent documents may be altered to change the locations of one or more hot spots relative to the antenna. Examples of such embodiments include embodiments wherein the hot spots are brought closer to a vent or to a proximal portion of the antenna. In one such embodiment, the largest hot spot, is located less than half the length of the antenna from the proximal end of the antenna.

Any of the antennas disclosed in the referenced patent documents may be altered to increase the size of the antenna or an electromagnetic near field of the antenna to reduce the hot spot.

Any of the antennas disclosed in the referenced patent documents may be adjusted before or during use. Examples of such methods include, but are not limited to: mechanically adjusting one or more portions of an antenna, changing the shape of an antenna based on a user input, changing the shape of an antenna based on heat (e.g. of nitinol based antennas by nitinol relaxation), and using a multi circuit antenna.

### 3. Altering non-antenna portion(s) of a device.

This may be achieved by methods including, but not limited to one or more of the following: adjusting the positon of a sheath relative to an antenna, bringing one or more hot spots closer to a vent before/during/after energy delivery, circulating a cooling fluid in one or more portions of a device or tissue, creating a closed circuit heat pipe, introducing or withdrawing an accessory device or material, causing a heat based shape change (e.g. of nitinol based device), and adjusting the shape of a device portion. Examples of methods of adjusting the shape of a device portion include, but are not limited to: mechanically adjusting one or more portions of an device, adjusting the shape of a device based on a user input, adjusting the shape of a device based on heat (e.g. of nitinol based devices), and using a multi circuit device.

### 4. Cooling one or more device regions or region(s) surrounding an antenna

This may be achieved by methods including, but not limited to one or more of the following: introducing one or more fluids before/during/after energy delivery, cooling one or more device/tissue regions before/during/after energy delivery, moving a device/antenna to a cooler tissue region, and vibrating one or more device regions.

Any of the methods disclosed herein may be used to prevent or reduce vapor generation or accumulation around an antenna such that the dielectric effect of the vapor is reduced or eliminated thereby preventing a rise in RP. Energy delivery methods operating at lower RP allow more efficient energy delivery.

Several method. and device embodiments are disclosed herein based on the total energy dose delivered to tissue (Jt). In one embodiment, Jt is defined as the total energy dose emitted by an antenna or other energy delivery element during a procedure. In another embodiment, Jt is defined as the total energy dose absorbed by tissue during a procedure. Jt is different from the total energy dose sent to a device from a generator, since there may be several losses and reflections on the transmission path leading to the tissue. Jt is also better correlated to a tissue thermal effect from an energy delivery procedure than the energy dose emitted by the generator. In one embodiment of a microwave energy delivery method, Jt is calculated based on one or more of: 1. energy dose transmitted by a generator to a device, 2. expected losses (as shown in Fig. 1) and reflections during an energy delivery path to tissue, 3. one or more RP parameters, and 4. adjustments based on pre-procedure testing of one or more system components. Examples of system components include, but are not limited to: antennas, transmission lines, energy generators, and mechanical (non-electrical components). One or more Jt values may be displayed or otherwise communicated to a user before/during/after an energy delivery procedure and used to guide or otherwise control a procedure.

The system and/or the user may stop energy delivery if a desired Jt dose is reached. The system and/or the user may restart or continue energy delivery if a desired minimum Jt dose is not reached. After an energy delivery procedure, the calculated Jt may be used to predict the efficacy and/or the safety of an energy delivery procedure.

In one embodiment, an antenna is moved while delivering energy. In one such embodiment, one or more motion parameters (e.g. decision whether to move the antenna, total displacement, rate of motion, direction of motion, etc.) are based on a Jt or RP parameters disclosed herein.

In one embodiment, an antenna or other energy delivery element is moved to extend a previously created lesion. Examples of such extension methods are disclosed in US patent US9462642. In one such embodiment, one or more extension parameters (e.g. decision whether to extend the lesion, total antenna displacement, rate of antenna motion, direction of displacement, etc.) are based on a Jt or RP parameters disclosed herein.

In one embodiment, an energy delivery system and/or a user create a desired thermal effect by delivering a particular It dose. In one such embodiment, a desired ablation depth is achieved by delivering a pre-determined Jt dose. The energy delivery system may have an upper limit of Jt to avoid potential adverse events or a lower limit of Jt to prevent an inefficacious procedure. In one embodiment, a user is provided with data regarding the correlation of Jt dose and the thermal effect (e.g. an ablation depth) or a clinical outcome. Thereafter, the user selects an appropriate Jt dose and delivers that dose to tissue to achieve the desired ablation depth. The data may be provided as a table, a graph or other data structure. The Jt dose may be personalized or modified by a system or a user based on one or more input variables. In one embodiment, a Jt dose is build up in a modular fashion based on one or more input variables.

Examples of parameters that may be used to calculate or otherwise adjust or tailor Jt or the desired thermal effect in any of the embodiments herein include, but are not limited to: The presence/absence of blood and other fluids; perfusion characteristics of blood and other fluids; a measured or desired device and/or tissue temperature level; desired procedure time; intra-operative or preoperative measurements: where examples of such measurements include, but are not limited to: RP, impedance, temperatures, patient's pain level, detection of a condition potentially compromising the safety of a procedure, etc.; desired safety of an energy delivery procedure: Jt may be lower if an additional level of safety is desired; desired efficacy of an energy delivery procedure: Jt may be higher if an additional level of efficacy is desired; physician's preference(s) or input(s), type of anesthesia: In one such embodiment, the Jt may be lower for procedures performed under lower levels of anesthesia; cycle phase: In one such embodiment, Jt is higher during a biological cycle (e.g. menstrual cycle) when the target region (e.g. the endometrium) is thicker; tissue type; tissue pretreatment: Examples of pretreatments include, but are not limited to: hormonal or other medical pretreatment, surgical pretreatment (e.g. excision, scraping, curettage, cutting, etc.), and radiation treatment; the age of the patient; the patient's life stage; tissue location; and/or previous surgical procedures such as Cesarean sections.

Anatomical characteristics and dimensions can also be used to calculate or otherwise adjust or tailor Jt or the desired thermal effect in any of the embodiments herein. For example, one or more medical imaging methods including, but not limited to: endoscopy, ultrasound, MRI, fluoroscopy/CT/X-ray PET scans, etc. may be used. Examples of dimensions include, but are not limited to: lengths, widths, thicknesses, areas, volumes, etc. of one or more anatomical regions or tissues. For endometrial ablation procedures, anatomical characteristics and dimensions include one or more of: endometrial thickness, uterine cavity length, uterine cavity width, one or more uterine external size parameters, presence of fluid in the uterine cavity, distortions of the uterine walls or the uterine cavity e.g. fibroids, polyps, Müllerian anomalies, defects in the uterine wall, etc.

Pain outcomes and patient's level of pain tolerance can be used to calculate or adjust Jt and/or the desired thermal effect: Patients with higher pain tolerance may be treated with a higher Jt to maximize procedure efficacy. Patients with lower pain tolerance may be treated with the minimum Jt necessary to have a satisfactory outcome while avoiding excessive pain. One or more non-Jt energy delivery parameters (e.g. a power level) may also be adjusted before and/or during a procedure to reduce pain experienced by a patient. In one embodiment, menstrual cycle pain or labor pain are used as a marker for patient's level of pain tolerance. In one embodiment, the end point of an energy delivery procedure is based on pain. If the patient feels a defined level of pain, the procedure is terminated, or is deemed to be effective.

Jt can also be adjusted to account for a patient preference(s) or input(s) For example, a patient willing to tolerate a higher level of pain may be treated with a higher Jt dose and vice versa. After a certain minimum level of Jt is delivered, the procedure may be terminated based on a patient or physician's input. In another embodiment, the patient controls one or more energy delivery parameters (e.g. a power level, a duty cycle, etc.) based on the pain he is experiencing. The patient may be given a handheld or other control (e.g. a key pad) to adjust the energy delivery parameter(s). Alternately, the patient may dictate instructions to a user who then adjusts the energy delivery parameter(s).

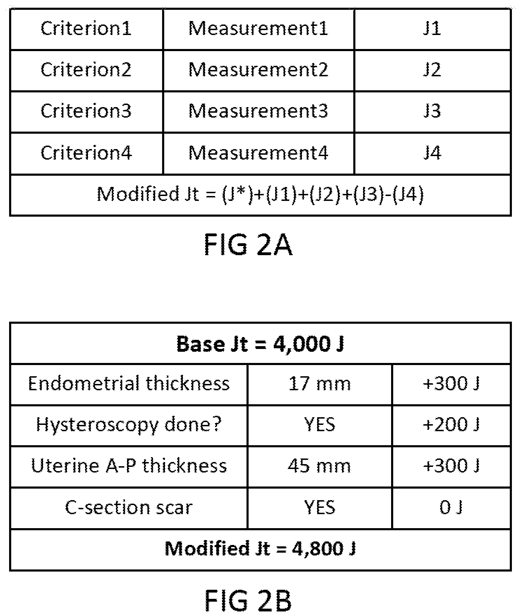

Figs. 2A - 2C show example tables of modular buildup of one or more Jt parameters. Fig. 2A shows a typical example of a modular buildup of a Jt parameter based on measurements or other inputs of one or more criteria. The measurement or other input of each criterion leads to an adjustment factor (J1,J2,J3,J4). The final Jt is calculated by adjusting a base J* as shown in the equation in Fig. 2A. Such adjusted Jt may be used for any of the method embodiments herein.

Fig. 2B shows an embodiment of a modular Jt buildup for an endometrial ablation procedure. In this embodiment, the base Jt is 4,000 J for energy delivery adjacent to the uterine fundus. In this embodiment, the base Jt is adjusted based on endometrial thickness (e.g. radiological measurement of the endometrial stripe), whether hysteroscopy was done before the ablation, uterine external thickness in the anterior-posterior direction, and whether the uterus has a C-section scar. Jt may be adjusted higher for higher endometrial thickness. It may be adjusted higher if hysteroscopy was done before the ablation. Jt may be adjusted higher for larger uteri. Jt may be adjusted higher if a C-section scar is absent. Other criteria that may be used include, but are not limited to one or more of sounding length (e.g. higher Jt for longer sounding length such as >8 cm), uterine cavity length (e.g. higher Jt for longer uterine cavities such as >5cm), uterine cavity width (e.g. higher Jt for wider uterine cavities such as >4cm), patient's age (e.g. higher Jt for younger patients such as patients younger than 44), presence of adenomyosis (e.g. higher Jt if patient has known or suspected adenomyosis), painful periods/dysmenorrhea (e.g. higher Jt for patients with more painful periods such as VAS scores >5), patient's pain tolerance and treatment setting (e.g. Jt may be adjusted lower if the patient's pain tolerance is lower or the treatment is being done in the outpatient setting), and endometrial thinning due to one or more of menopausal or post-menopausal status, drugs such as progestins, hormonal IUDs, any anatomical or patient parameter disclosed herein, etc.

The Jt parameter may be customized based on number and type of C-sections to prevent injury to non-target anatomy. Other methods of lowering the risk of an energy delivery procedure disclosed herein include, but are not limited to: lowering a power level, lowering a time limit, lowering a Jt value, lowering a RP limit, and stopping or halting a procedure before completion.

In any of the embodiments herein, one or more energy delivery parameters (e.g. Jt parameters) may be auto adjusted based on one or more inputs obtained during or before energy delivery.

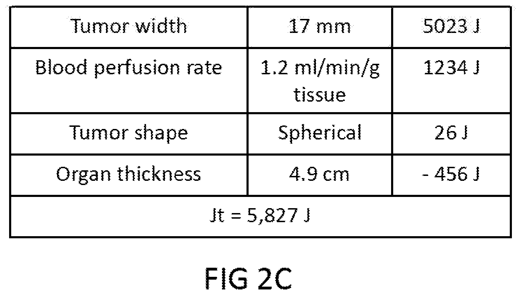

Fig. 2C shows an embodiment of a modular Jt buildup for a tumor ablation procedure. In this embodiment, the Jt is calculated from the tumor width, blood perfusion rate, tumor shape, and the thickness of the organ containing the tumor. Jt may be adjusted higher for larger tumors. Jt may be adjusted higher for higher blood perfusion rate. Jt may be adjusted higher for irregularly shaped tumors. Jt may be adjusted lower if the organ is smaller. Jt may be adjusted higher or lower depending on the risk versus benefit of a treatment Other criteria disclosed herein may also be used for adjusting the Jt.

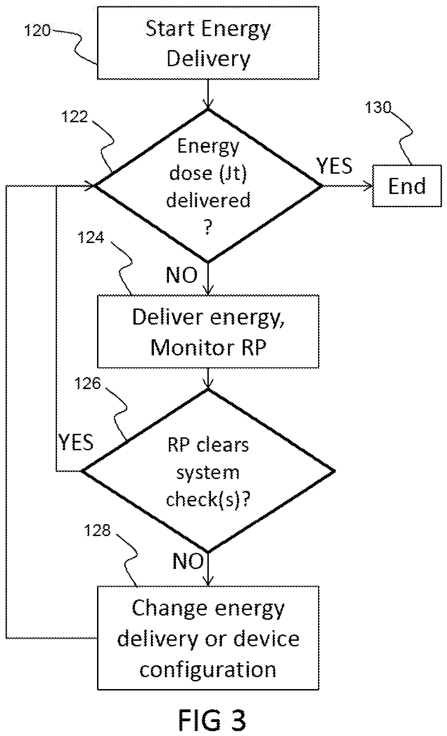

Fig. 3 shows an example of a process used in the present disclosure for delivering a Jt dose of microwave energy to tissue. At step 120, the energy delivery is started. At step 122, the system checks if the desired Jt dose has been delivered. The Jt dose can be set by a user or by a system using any of the methods and device embodiments disclosed herein. If the Jt dose has not been delivered, the method proceeds to step 124 and monitors a RP parameter. Examples of RP parameters have been disclosed elsewhere in this specification. Thereafter, at step 126, the system delivers energy and determines if the measured RP parameter clears one or more system checks. Examples of such system checks include, but are not limited to: check if RP parameter is under a limit (e.g. a measured RP is < 25%, 50%, etc.), check if RP parameter is over a limit, check if RP parameter is within a range, check if RP rise is under a limit (e.g. < 2% per second), check if the RP parameter does not exceed a limit for more than a particular time duration (e.g. if a RP parameter does not exceed 50% for more than 4 s), etc. One or more system checks may be used to check for one or more conditions disclosed herein including, but not limited to: vapor accumulation, desiccation of tissue, completion of an ablation procedure, mefficient energy delivery, disconnection of a component, etc. If the system checks are cleared, the method proceeds to step 122. If the system checks are not cleared in step 126, the method proceeds to step 128 wherein one or more energy delivery parameters and/or configuration of one or more device portions (including an antenna) is changed. Examples of energy delivery parameters and various methods of changing them are disclosed elsewhere in this specification. Examples of changing a configuration of one or more device portions (including an antenna) are disclosed elsewhere in this specification. At step 128, any of the methods disclosed herein for removing trapped vapor and/or preventing vapor from forming may be performed. At step 122, if the desired Jt has been delivered, the system proceeds to step 130 and ends energy delivery.

In one embodiment of the method shown in Fig. 3, Jt is 3,000 - 6,000 J. RP is measured at the antenna level as a fraction of the power reflected back to the antenna from tissue to the total forward power coming to the antenna from a transmission line. A system check comprises checking if RP does not exceed 50% +/-10% for more than 3 +/- 2 seconds. If this system check is not cleared, at step 128 energy delivery is paused and resumed after the pause.

In any of the embodiments herein, before an energy delivery procedure, one or more test pulses are sent to tissue and the patient's pain response is noted to the test pulses. The test pulses may have differing energy delivery parameters, examples of which are disclosed elsewhere in this specification. Particular examples include, but are not limited to: power level, energy delivery duration, size/shape of an energy delivery pulse, duty cycle, and energy dose. Thereafter, initial treatment or energy delivery parameters are selected and the treatment is started. Subsequent energy delivery may be modified based on any of the embodiments disclosed herein, The energy delivery may be terminated based on one or more Jt parameters as disclosed in this specification.

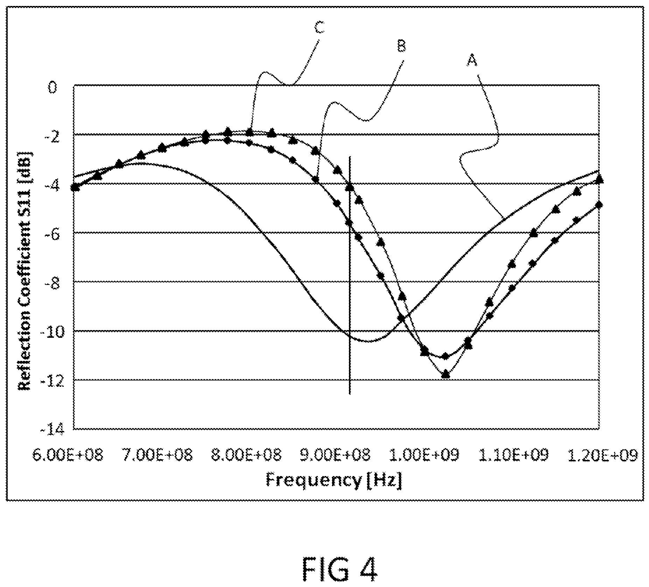

Fig. 4 shows graphs of reflection coefficients (S-parameter S.sub.11) of an antenna at three different time periods during an ablation procedure. The system delivers microwave energy at 915 MHz ISM band. Graph A shows the reflection coefficient at the start of the energy delivery procedure showing a good matching (around -10 dB) at 915 MHz. Graph B shows the reflection coefficient after the start of the energy delivery procedure wherein the matching has worsened and is now around -6 dB. The matching may worsen due to one or more energy delivery based situations disclosed herein including, but not limited to: accumulation of vapor/steam around an antenna and desiccation of tissue. Graph C shows the reflection coefficient well into the energy delivery procedure wherein the matching has further worsened and is now around -4 dB. Again, the matching may worsen due to one or more energy delivery based situations disclosed herein including, but not limited to: accumulation of vapor/steam around an antenna and desiccation of tissue. Thereafter, using one or more of the methods and devices disclosed in this invention, the reflection coefficient (S-parameter S.sub.11) of an antenna is brought as close to Graph A as possible. During an energy delivery procedure, one or more measurements of the reflection coefficient of an antenna may be used to perform any of the methods disclosed herein.

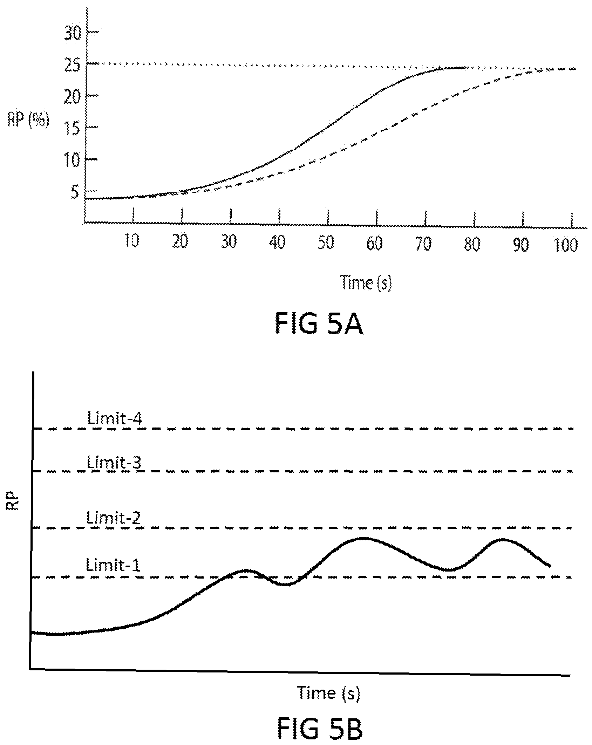

Fig. 5A shows a comparison of two RP curves with and without using the methods disclosed herein respectively. Fig. 5A shows a graph of RP values during an energy delivery procedure against time. A first curve (solid line) is from a system that does not incorporate an embodiment of the present invention. The RP rises with time due to one or more conditions disclosed herein (e.g. vapor accumulation, desiccation of tissue, completion of an ablation procedure, etc.) and reaches an RPL of 25% in about 78 s. A second curve (dashed line) is from a system that incorporates an embodiment of the present invention wherein an energy delivery parameter is changed in response to rise in RP. This causes the RP rise to slow down (e.g. by reducing vapor accumulation) and thus RPL is reached much later at 100 s. This allows the system to deliver a higher energy dose to tissue.

Fig. 5B shows a graph of RP relative to time using a system of the present invention comprising four RP limits. A first RPL "Limit-1" is used to determine if the energy delivery is inefficient and acts as a trigger for modification of energy delivery as disclosed elsewhere in this specification. A second RPL "Limit-2" is used to determine if the energy delivery is more inefficient and must be terminated or altered immediately or sooner. Third and fourth RPL "Limit-3" and "Limit-4" respectively are used to determine if a component (e.g. an antenna, a transmission line, a connector) of the system is broken or disconnected. The system shuts down energy delivery if the RP exceeds "Limit-2", "Limit-3", and "Limit-4" levels for a pre-determined time. However, the pre-determined times may be different for the three RPLs. In one embodiment, Limit-1 is 50% and RP is measured at the antenna level as a fraction of the power reflected to the antenna from tissue to the total forward power from the antenna to the tissue. The system uses any of the methods disclosed herein to change energy delivery once RP exceeds 50% and shuts down energy delivery once RP exceeds 50% for more than 4 +/- 3 seconds. As the graph in Fig. 4B shows, during an energy delivery procedure, the RP rises due to factors disclosed herein. As the RP crosses Limit -1 for the first time, the user and/or the system change energy delivery which in turn causes RP to fall below Limit-1. Thereafter, RP rises again with more energy delivered and crosses Limit-1 for the second time. Thereafter, the user and/or the system again change energy delivery which in turn causes RP to fall, but remain above Limit-1. This process may be repeated to keep the RP between Limit-1 and Limit-2.

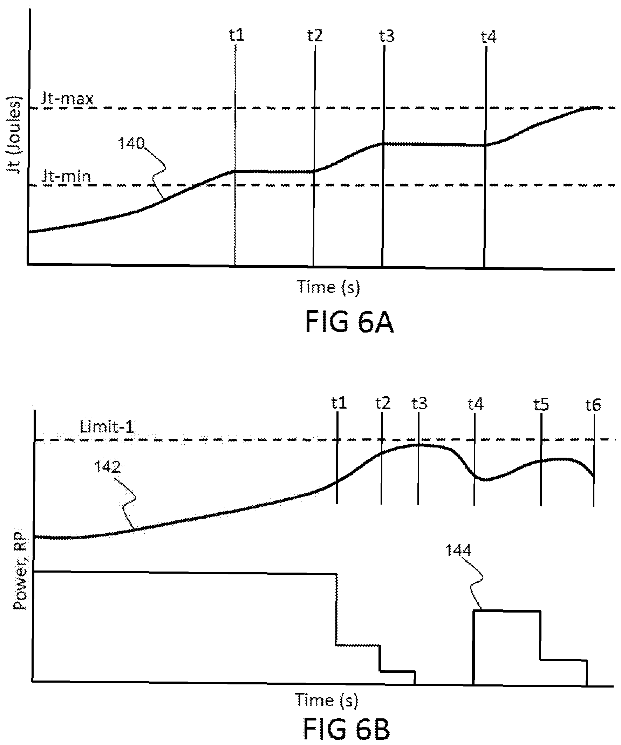

Fig. 6A shows a graph of Jt versus time for an energy delivery procedure comprising multiple pauses. In Fig. 6A, graph 140 of Jt rises with time until it crosses a Jt-min level as shown. At time t1, the Jt (or any other energy delivery parameter such as RP disclosed herein) meets one or more criteria listed herein which causes the system to pause energy delivery at time t1. During the pause, one or more actions (e.g. venting) listed herein may be performed. During the pause, one or more effects listed herein (e.g. condensation of steam/vapor, rehydration of tissue, etc.) may take place. Thereafter the user and/or the system restarts energy delivery at time t2 as shown Energy delivery proceeds until time 13, when the Jt (or any other energy delivery parameter such as RP disclosed herein) meets one or more criteria listed herein which causes the system to pause energy delivery at time t3. During the pause, one or more actions (e.g. venting) listed herein may be performed. During the pause, one or more, effects listed herein (e.g. condensation of steam/vapor, rehydration of tissue, etc.) may take place. Thereafter the user and/or the system restarts energy delivery at time t4 as shown. Energy delivery proceeds until the Jt reaches Jt-max as shown when the user and/or the system terminate energy delivery.

Multiple energy targets may be used to determine or create a variety of situations. The embodiment in Fig. 6A shows two energy targets (Jt-min and Jt-max). Multiple embodiments are possible using one or more method and device features disclosed herein to determine one or more of: antenna deployment, antenna positioning, completion of an energy delivery procedure, temperature of tissue, presence/absence of a potentially hazardous situation, etc. Multiple embodiments are possible using one or more method and device features disclosed herein to create one or more of: desired clinical effect (e.g. hypennenorrhea, amenorrhea, etc.), clinical effect in presence of a condition (e.g. creating a successful endometrial ablation in patient with adenomyosis), enhancing the safety of a procedure (e.g. delivering lower Jt for endometrial ablation in patients close to menopause or with thinner endometrial layers, etc.), reducing the pain experience by the patient undergoing an energy delivery procedure, etc.

Fig. 6B shows graphs of RP and power versus time for an energy delivery procedure comprising multiple changes in energy delivery parameters. In Fig. 6B, energy delivery starts are a power level as shown in graph 144. The energy delivery to tissue causes the RP to rise as seen in graph 142. At time t1, the RP level meets one or more criteria listed herein and at least one energy delivery parameter is modified. In the embodiment shown, the power level is reduced. The treatment progresses till time t2 with a further rise in RP. At time t2, the RP level meets one or more criteria listed herein and at least one energy delivery parameter is modified. In the embodiment shown, the power level is reduced further. The treatment progresses till time t3 with a further rise in RP such that the RP reaches close to RP Limit-1. Based on that, at time t3, at least one energy delivery parameter is modified. In the embodiment shown, the energy delivery is mined off. This causes RP to fall until time t4 when energy delivery is turned back on by the system and/or the user at time t4 using any of the embodiments disclosed herein. The treatment progresses till time t5 with a further rise in RP. At time t5, the RP level meets one or more criteria listed herein and at least one energy delivery parameter is modified. In the embodiment shown, the power level is reduced. Thereafter, the treatment progresses till time t6 when the energy delivery is terminated after meeting one or more termination criteria, examples of which are disclosed herein.

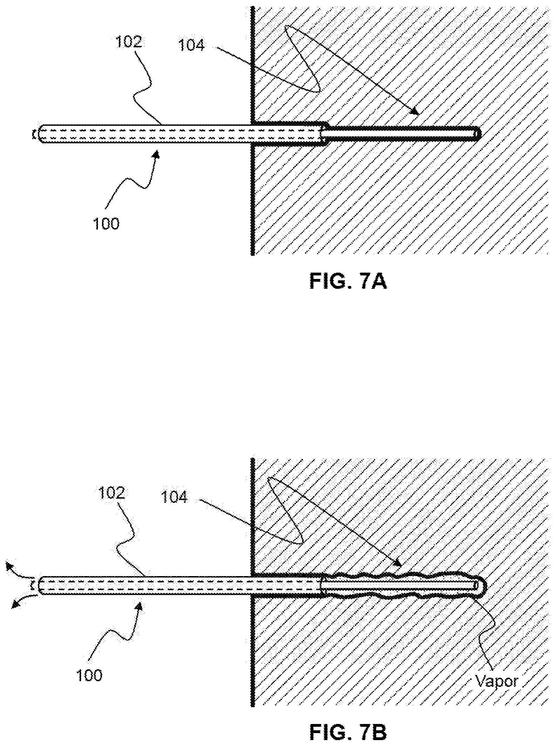

Figs. 7A and 7B an illustration comprising venting one or more products generated because of energy delivery. In Fig. 7A, an energy delivery device 100 comprising an antenna or other energy delivery element 104 is used to deliver energy to a target region. Device 100 comprises a sheath or cannula 102. A space or a lumen within cannula 102 or other regions of device 100 is in fluid communication with the target region to which energy is being delivered. The space or lumen may be between the inner surface of cannula 102 and the outer surface of a device component e.g. an antenna or a transmission line.

The space or lumen may be used to; evacuating or venting the products (e.g. steam, vapor, tissue debris, tissue fluid, saline, blood, plasma, gases, etc.) generated during an energy delivery procedure. Fig. 7B shows vapor generated during the energy delivery procedure being vented to the atmosphere. Any of the method and device steps disclosed herein may be used during the method shown in Figs. 7A and 7B. For example, rise in RP may be used to determine accumulation of gases in the anatomical region during a microwave energy delivery procedure. One or more methods of evacuating fluids may be employed. One or more methods of preventing or reducing vapor accumulation may be employed.

The space or lumen may also be used for one or more of: introducing one or more fluids or materials into the anatomy, evacuating fluids or other materials from an anatomical region; introducing liquids inside an anatomical region such as anesthetics, contrast agents, cauterizing agents, alcohols, thermal cooling agents, thermal heating agents, a fluid dielectric medium that surrounds antenna 104, antibiotics and other drugs, saline and flushing solutions, distension media; introducing gases for detecting perforation of organs such as the uterus, and applying suction to collapse an anatomical region around the antenna 104 or to evacuate products from an anatomical region. Suction may be applied in the uterine cavity to increase the contact of antenna 104 with the uterine endometrium. When a gas such as carbon dioxide is used for distending an anatomical region and/or for detecting perforation of an anatomical region, the gas may be delivered at a pressure between 20-200 mm Hg. In one embodiment, a fluid inserted into a bodily cavity that alters the local environment around cannula 102 and/or working device 104. In one such embodiment, a fluid (e.g. a liquid or gas) is introduced around one or more regions of working device 104 comprising a microwave or radiofrequency antenna. The fluid environment may alter (e.g. improve or worsen) the matching between the antenna and the surrounding target material. The level and/or the change in the microwave returned power with the amount of alteration of the local environment around antenna 104 may be measured and used to take further decisions. Examples of distension media include, but are not limited to: gases such as carbon dioxide and nitrogen; ionic liquids (e.g. saline solutions, lactate solutions); and non-ionic liquids (e.g. solutions of one or more of: sorbitol, glycine, dextrose, dextran, and mannitol). The distension medium may have a greater viscosity than that of water to reduce the leakage rate. The distension medium may be introduced in the anatomy through any of the fluid or device transport paths disclosed herein.

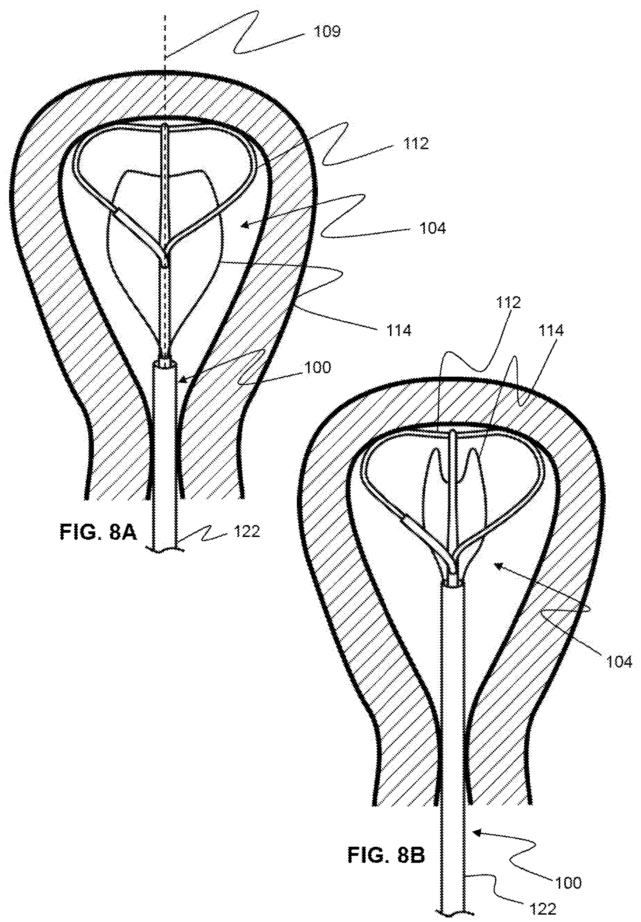

Figs. 8A and 8B show venting energy delivery products from the anatomy by moving one or more device regions. In Fig. 8A, an antenna 104 is used to deliver energy to tissue. Although in the embodiment shown, antenna 104 comprises radiating element 112 and shaping element 114 and is similar to antennas disclosed in US patent publications US20100121319A1 and US patent US8968287 , this method may be used with other antennas or energy delivery elements. Antenna 104 is introduced through cannula 122. There is a lumen or a space within the interior of cannula 122. In Fig. 8B, without substantially changing the position of antenna 104, cannula 122 is advanced over antenna 104 such that it covers at least a portion of antenna 104. In one embodiment, this step is used to create or clear a vent to the anatomical region by displacing one or more of: regions of device 100, one or more portions of antenna 104, tissue around antenna 104, fluids, and ablated or otherwise thermally modified tissue or tissue debris around antenna 104. In an alternate embodiment, without substantially changing the position of cannula 122, antenna 104 is withdrawn within cannula 122 such that cannula 122 covers at least a portion of antenna 104. In another embodiment, both antenna 104 and cannula 122 are displaced or moved. The motions disclosed in this embodiment are not limited to linear motions, but can also be rotations, vibrations, curvilinear motions, etc.

In one embodiment of a perforation detection test to rule out procedure related or naturally-present perforations of an anatomical region, a fluid is introduced into the anatomical region and leakage of fluid from the anatomical region is measured or detected. The leakage may be detected by one or more methods including, but not limited to: sensing flow, sensing pressure change, sensing volume change, sensing change in a size or shape parameter of a cavity, and sensing change in the distension of a cavity.

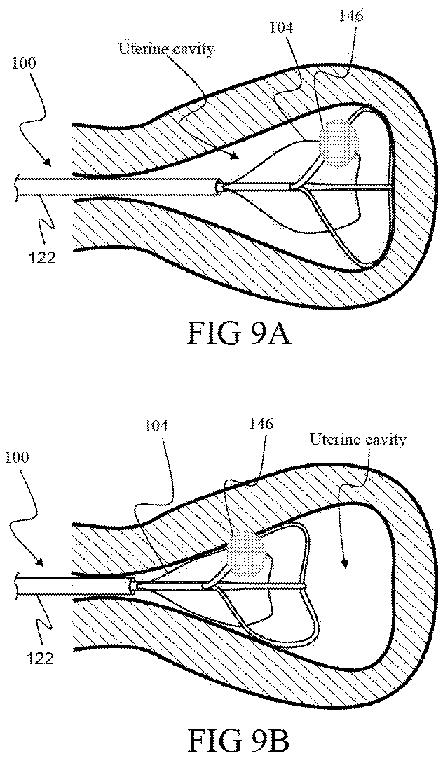

Figs. 9A and 9B show an embodiment of venting energy delivery products from the anatomy by altering the position of a hot spot of an antenna. In Fig. 9A, an antenna 104 comprising a hot spot 146 is used to deliver energy to tissue. A hot spot is defined as a region of concentrated electromagnetic field on an antenna. Although in the embodiment shown, antenna 104 comprises radiating element 112 and shaping element 114 and is similar to antennas disclosed in US patent publication US20100121319A1 and US Patent US8968287 , this method may be used with any antenna or energy delivery element having a hot spot. Antenna 104 is introduced through cannula 122. In Fig. 9B, antenna 104 is moved to a different tissue location and is used to deliver energy to the different tissue location such that the location of the hot spot is changed. This movement may be of any type disclosed herein. This movement may be made in response to any of the criteria and/or conditions disclosed herein.

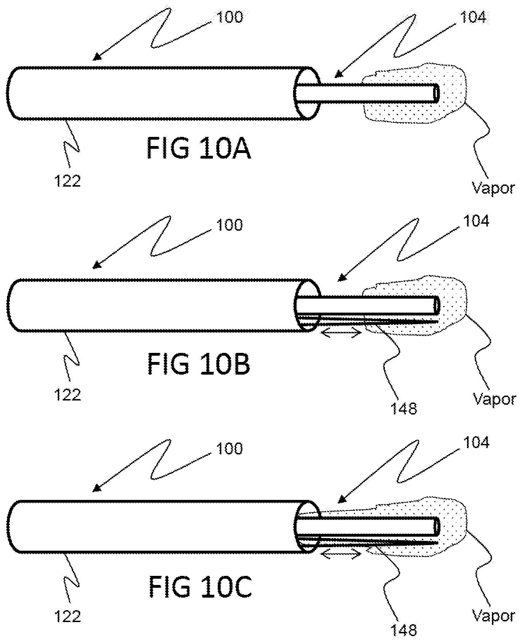

Figs. 10A - 10C show the steps of a method of creating or opening a vent for venting energy delivery products from the anatomy. In Fig. 10A, an antenna 104 is used to deliver energy to tissue. This energy delivery creates a pocket of fluids (e.g. vapor) generated during the energy delivery. However, the vapor is trapped in a pocket and is not vented. This may lead to a rise in RP in a microwave energy delivery procedure and consequent inefficient energy delivery. In the step shown in Fig. 10B, a venting device 148 is introduced into the anatomy such that at least a portion of venting device 148 is near or inside the vapor pocket. Examples of venting devices include, but are not limited to: curved lengths of hollow or non-hollow medical grade materials, hollow structures, stylets, devices comprising one or more loops, devices comprising means for applying suction, etc. In the embodiment shown, venting device 148 comprises a loop that is inserted into the air pocket. Thereafter, venting device 148 may be rotated and/or translated and/or otherwise moved to connect the vapor pocket to a vent. This may be achieved by displacing one or more of: fluids, regions of deice 100, tissue regions or pieces, and debris generated during energy delivery. In the step shown in Fig. 10C, the vapor pocket is evacuated through a vent (e.g. the same lumen through which venting device is introduced).

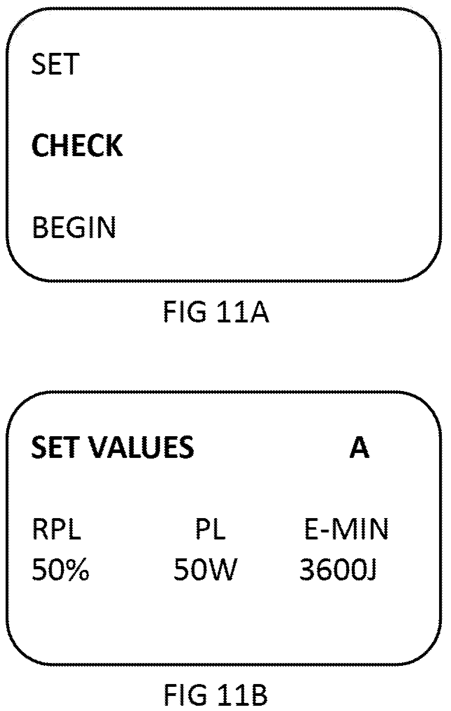

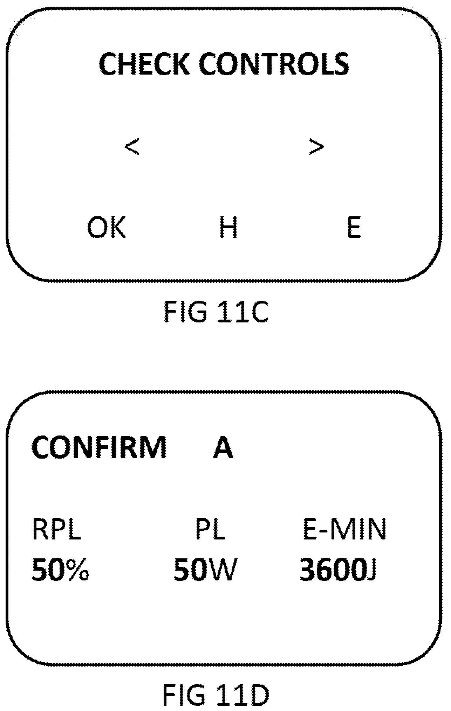

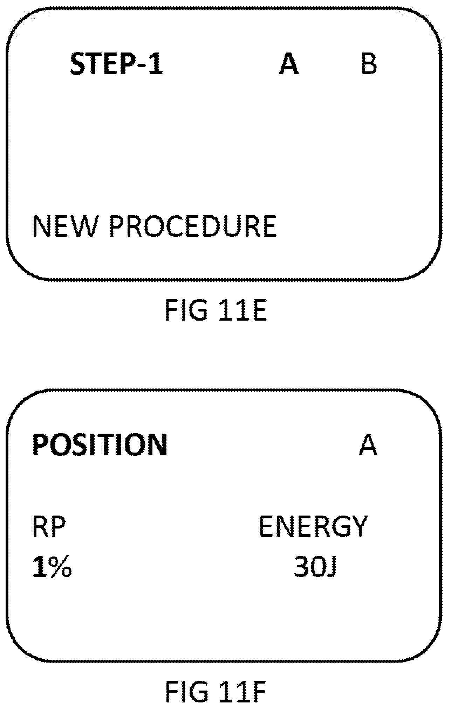

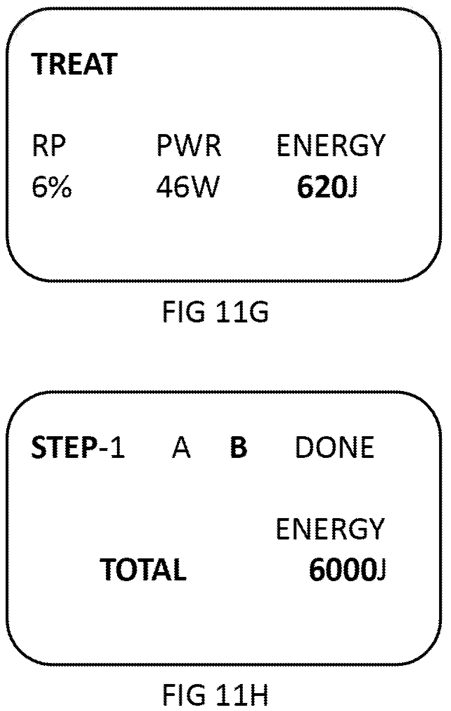

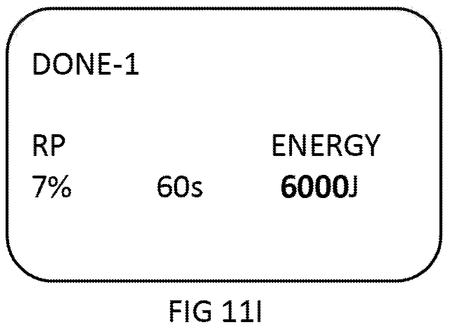

Figs. 11A - 11I shows views of a display screen during one embodiment of a microwave energy delivery procedure. In this embodiment, the user selects one of the three options from a "Home Screen" shown in Fig. 11A: SET, CHECK, and BEGIN. In the SET option, the user can select one or more of: 1. a returned power limit shown as "RPL", 2. a treatment power shown as "PL" (e.g. power level at the end of a transmission line terminating in an antenna) and an "E-min", the minimum energy dose to be delivered from an antenna to tissue. These three parameters may be stored as treatment modes (an example Mode A is shown in Fig. 11B) i.e. a set of treatment parameters stored in the system. In one embodiment, an "E-max" (the maximum energy dose to be delivered from an antenna to tissue in a particular mode) may be set into the system and may not be changeable by the user for ensuring patient safety. In another embodiment, E-max can be adjusted or changed by the user. In the SET option, the user may also select or determine system volume, language, software version, etc. In the CHECK option, the user selects one or more of: checking proper functioning of an energy delivery/generation portion such as a microwave generator, checking proper functioning of a cable connecting a generator to a device or other cables in a system, check proper functioning of one or more buttons (e.g. scroll buttons, selection buttons, home screen button, energy delivery buttons, etc.) on a keypad or other input device (example screen shown in Fig. 11C), a display, etc. In one embodiment, when the user selects the BEGIN, option, a confirmation screen e.g. a "CONFIRM A" screen as shown in Fig. 11D is displayed for the user to confirm the settings of Mode A. If the settings are correct, the user proceeds to the next step. More confirmation screens may be similarly displayed. Once the user confirms the proper parameters were selected, the system displays the option to select and use one of the stored modes as shown in Fig. 11E. When the user selects a mode, the system displays the option to deliver a test dose of energy in the POSITION screen. One or more RP measurements are made during the test dose energy delivery as shown in Fig. 11F. Further, an energy delivery counter is started to track the total energy delivered by the antenna to tissue as shown in FIG 11F. The RP measurements may be used for one or more of the functions disclosed herein and in US Patent US9462642, the entire disclosure of which is incorporated herein by reference. After the user is satisfied with the RP measurements, the user selects the next screen called a TREAT screen (shown in Fig. 11G) and starts the energy delivery. During energy delivery, the system measures the RP and displays the power being delivered to the tissue "PWR", and the total energy dose delivered to tissue "ENERGY". The power may be changed by the user during energy delivery. The system may proceed to pause the energy delivery is the RP meets a defined criterion (e.g. RP => RPL for >3 seconds), if the power delivery from antenna to tissue is too low or other changes disclosed herein, etc. After pausing, the user may restart the energy delivery. Before restarting, the user may perform any of the methods disclosed herein or may simply wait for some time. The energy delivery may be terminated for a mode if one or more of the following conditions are met: E-min and RPL are both reached, E-min is reached and the power delivery from antenna to tissue is too low, E-max is reached, or a time limit is reached. Thereafter, the user may select a new mode e.g. Mode B to deliver energy (e.g. for an Extension treatment to extend a previously created thermal effect), or may end the patient treatment or start the treatment for a new patient through the screen shown in Fig. 11H. Once a patient's treatment is completed, the system may display a summary of the treatment e.g. total energy delivered to the patient, energy delivered in particular modes, energy delivery time (total and per mode, etc.), etc. An example of a summary screen is shown in Fig. 11I. In one embodiment, the system displays an audio signal e.g tone or a beep, to indicate that energy delivery is on. The audio signal may change one or more times during an energy delivery procedure to indicate one or more of: power level to tissue, E-min reached or not reached, E-max reached, RPL reached, RP too high, or any other condition disclosed herein.

In any of the embodiments herein, notifications/conununications regarding one or more aspects of an energy delivery procedure may be displayed or otherwise communicated to the user regarding one or more of: tissue or energy delivery conditions e.g. vapor accumulation, ablation progress, inefficient energy delivery, etc.; procedure status e.g. percent energy delivery completed, amount remaining, energy delivery not complete, etc.; energy parameters e.g. total energy delivered, total energy remaining to be delivered, set dose, etc.; time parameters e.g. estimated time remaining, countdown clock, etc.; system performance normal; ok for a user to terminate procedure e.g. after reaching E-min; prompt to perform any of method steps e.g. any of the method steps disclosed herein, error conditions e.g. e.g. low level of power being delivered by antenna to tissue, breakage and/or disconnection of one or more components, inconect settings selected, a physical parameter such as temperature or an energy parameter being out of a limit, etc.; patient counselling/reassurance e.g. of one or more of: treatment progressing as per the plan, expected level of safety or efficacy outcomes of an energy delivery procedure, estimated time remaining until procedure completion, etc.; user selection has reached the end of range, and prompt about procedure steps e.g. move sheath, engage a vent, change a device configuration, change a treatment parameter, etc.

Any of the notifications/communications above may be performed using visual, audio or audiovisual signals. Audio signals may be one or more of: beeps, short beeps, long beeps, double beeps, triple beeps,6-beeps, alert beeps, etc. One or more parameters such as frequency, duration, etc. of the beeps or other audio signals may be modified to indicate one or more energy delivery parameters. Examples of such parameters include, but are not limited to: power being delivered to tissue, E-min reached, E-max reached, E-min not reached, E-max not reached, paused energy delivery, cancelled energy delivery, error condition(s), etc.

Various additional features may be present on or added to the devices disclosed herein to confer additional properties to the devices disclosed herein. Examples of such features include, but are not limited to one or more lumens, ability to apply a vacuum or suction to the target anatomy, ability to visualize one or more regions of the target anatomy, ability to limit the depth of insertion into the target anatomy, ability to deploy the antenna, ability to connect to a source of energy, etc.

In any of the embodiments disclosed herein, a medical procedure using antenna 104 may be terminated or prevented from starting if the position and/or deployment of antenna 104 and/or device 100 are improper. This may be done by one or more of: preventing energy delivery through antenna 104, shutting down a mechanical or electrical function of device 100, and informing the user about improper position and/or deployment of antenna 104 and/or device 100.

While variations of the system and method are described using the uterus as an example of a target, endometrial ablation procedures as examples of energy delivery methods, microwave energy as an example of energy, and returned power as an example of losses during energy delivery. However, these have been used as examples only and the invention is not limited to these specific examples. For example, losses during energy delivery may occur from one or more of: radiation, dielectric heating, conduction, convection, reflections, and steam/vapor generation.

Any of the generators disclosed herein may comprise one or more of: a processor, a memory, and a user interface for performing one or more of the method embodiments disclosed herein. The methods and systems described herein can be used in any region of the body and on any tissue or organ.

Any of the generators disclosed herein may comprise one or more of: a processor, a memory, and a user interface.

The scope of the invention is defined by the appended claims. Several examples or embodiments of the invention have been discussed herein, but various modification, which fall under the scope of the claims, may be made.

## Claims

1. A system for applying energy through an interconnecting cable, ICC, to an energy delivery device (100) including an antenna (104) for delivering microwave energy to tissue, the system comprising:
a source of energy;
a memory unit configured to store at least one target energy dose;
a user interface, where the user interface is configured to increase or decrease the at least one target energy dose comprising a maximum target energy dose and a minimum target energy dose; and
a controller configured to apply an applied energy dose to the energy delivery device from the source of energy,
**characterized in that** the controller is configured
to calculate an energy loss during energy delivery comprising returned power measured at the antenna as a fraction of the power reflected back to the antenna from tissue to the total forward power from the antenna to tissue, wherein returned power measurements include measurements of returned power magnitude, phase and/or ratio of the returned power to the incident power, where the controller is further configured to determine an amount of delivered energy using the energy loss and to compare the amount of delivered energy to the at least one target energy dose to provide a signal when the applied energy dose reaches the at least one target energy dose, wherein the controller is further configured to stop energy delivery when the delivered energy dose is greater than the minimum target energy dose and the returned power is greater than a desired returned power.

2. The system of claim 1, where the user interface is configured to display one or more patient data associated with increasing or decreasing the at least one target energy dose.

3. The system of claim 2, where the one or more patient data comprises data selected from the group consisting of an anatomic feature, an anatomic measurement, a previous medical procedure, a medical condition, and a lower pain tolerance.

4. The system of claim 1, where the user interface is configured to display an alert when the applied energy dose is greater than the minimum target energy dose.

5. The system of claim 1, where the user interface is configured to display one or more losses during energy delivery.

6. The system of claim 1, where the user interface is configured to display one or more energy parameters during energy delivery.

7. The system of claim 6, wherein the energy parameter is the total energy dose delivered to tissue, and is calculated from the value of a returned power.

8. The system of claim 1, wherein the loss during energy delivery comprises one or more losses selected from the group consisting of: radiation, dielectric heating, conduction, convection, reflection, and steam or vapor generation.

9. The system of claim 1, where the signal is configured to alter application of the applied energy dose.

10. The system of claim 1, where the memory unit is further configured to store at least one energy delivery parameter.

11. The system of claim 1, where the source of energy is configured to cause the energy delivery device to produce a thermal effect.

12. The system of claim 1, further comprising means to allow a user to change an energy delivery parameter during use.

## Patentansprüche

1. System zum Anlegen von Energie durch ein Verbindungskabel, ICC, an eine Energiezufuhrvorrichtung (100), die eine Antenne (104) zur Zufuhr von Mikrowellenenergie an Gewebe beinhaltet, wobei das System aufweist:
eine Energiequelle;
eine Speichereinheit, die derart konfiguriert ist, dass sie zumindest eine Ziel-Energiedosis speichert;
eine Benutzerschnittstelle, wobei die Benutzerschnittstelle derart konfiguriert ist, dass sie die zumindest eine Ziel-Energiedosis, umfassend eine maximale Ziel-Energiedosis und eine minimale Ziel-Energiedosis, erhöht oder verringert; und
eine Steuerung, die derart konfiguriert ist, dass sie eine angelegte Energiedosis an die Energiezufuhrvorrichtung von der Energiequelle anlegt,
**dadurch gekennzeichnet, dass**
die Steuerung derart konfiguriert ist, dass sie einen Energieverlust während der Energiezufuhr berechnet, umfassend eine Rückleistung, die an der Antenne als ein Bruchteil der Leistung gemessen wurde, die von dem Gewebe zur Antenne zur gesamten Vorwärtsleistung von der Antenne zum Gewebe zurückreflektiert wird, wobei die Rückleistungsmessungen Messungen der Größe der Rückleistung, der Phase und/oder des Verhältnisses der Rückleistung zur Einfallleistung beinhalten, wobei die Steuerung ferner derart konfiguriert ist, dass sie eine Menge der zugeführten Energie unter Verwendung des Energieverlusts bestimmt und die Menge der zugeführten Energie mit der zumindest einen Ziel-Energiedosis vergleicht, um ein Signal bereitzustellen, wenn die angelegte Energiedosis die zumindest eine Ziel-Energiedosis erreicht, wobei die Steuerung ferner derart konfiguriert ist, dass sie die Energiezufuhr stoppt, wenn die zugeführte Energiedosis größer als die minimale Ziel-Energiedosis ist und die Rückleistung größer als eine gewünschte Rückleistung ist.

2. System nach Anspruch 1, wobei die Benutzerschnittstelle derart konfiguriert ist, dass einzelne oder mehrere Patientendaten angezeigt werden, die dem Erhöhen oder Verringern der zumindest einen Ziel-Energiedosis zugeordnet sind.

3. System nach Anspruch 2, wobei die einzelnen oder mehreren Patientendaten Daten umfassen, die ausgewählt werden aus der Gruppe bestehend aus einem anatomischen Merkmal, einer anatomischen Messung, einem früheren medizinischen Verfahren, einem medizinischen Zustand und einer geringeren Schmerztoleranz.

4. System nach Anspruch 1, wobei die Benutzerschnittstelle derart konfiguriert ist, dass sie eine Warnung anzeigt, wenn die angelegte Energiedosis größer als die minimale Ziel-Energiedosis ist.

5. System nach Anspruch 1, wobei die Benutzerschnittstelle derart konfiguriert ist, dass sie einen oder mehrere Verluste während der Energiezufuhr anzeigt.

6. System nach Anspruch 1, wobei die Benutzerschnittstelle derart konfiguriert ist, dass sie einen oder mehrere Energieparameter während der Energiezufuhr anzeigt.

7. System nach Anspruch 6, wobei der Energieparameter die gesamte Energiedosis ist, die an das Gewebe zugeführt wird, und aus dem Wert einer Rückleistung berechnet wird.

8. System nach Anspruch 1, wobei der Verlust während der Energiezufuhr einen oder mehrere Verluste umfasst, die ausgewählt werden aus der Gruppe bestehend aus Strahlung, dielektrischer Erwärmung, Leitung, Konvektion, Reflexion und Dampf- oder Wasserdampferzeugung.

9. System nach Anspruch 1, wobei das Signal derart konfiguriert ist, dass es die Anwendung der angelegten Energiedosis verändert.

10. System nach Anspruch 1, wobei die Speichereinheit ferner derart konfiguriert ist, dass sie zumindest einen Energiezufuhrsparameter speichert.

11. System nach Anspruch 1, wobei die Energiequelle derart konfiguriert ist, dass sie die Energiezufuhrvorrichtung dazu veranlasst, einen thermischen Effekt zu erzeugen.

12. System nach Anspruch 1, ferner umfassend Mittel, die es einem Benutzer ermöglichen, einen Energiezufuhrsparameter während der Benutzung zu ändern.

## Revendications

1. Système d'application d'énergie par le biais d'un câble d'interconnexion, ICC, à un dispositif de distribution d'énergie (100) incluant une antenne (104) permettant de distribuer une énergie à micro-ondes à un tissu, le système comprenant :
une source d'énergie ;
une unité de mémoire configurée pour stocker au moins une dose d'énergie cible ;
une interface utilisateur, où l'interface utilisateur est configurée pour augmenter ou réduire l'au moins une dose d'énergie cible comprenant une dose d'énergie cible maximale et une dose d'énergie cible minimale ; et
un dispositif de commande configuré pour appliquer une dose d'énergie appliquée au dispositif de distribution d'énergie à partir de la source d'énergie,
**caractérisé en ce que**
le dispositif de commande est configuré pour calculer une perte d'énergie pendant une distribution d'énergie comprenant une puissance retournée mesurée au niveau de l'antenne en tant que fraction de la puissance réfléchie vers l'antenne à partir d'un tissu sur la puissance vers l'avant totale de l'antenne vers le tissu, dans lequel des mesures de puissance retournée incluent des mesures de magnitude de puissance retournée, de phase et/ou de ratio de la puissance retournée sur la puissance incidente, où le dispositif de commande est en outre configuré pour déterminer une quantité d'énergie distribuée au moyen de la perte d'énergie et pour comparer la quantité d'énergie distribuée à l'au moins une dose d'énergie cible pour fournir un signal lorsque la dose d'énergie appliquée atteint l'au moins une dose d'énergie cible, dans lequel le dispositif de commande est en outre configuré pour mettre fin à la distribution d'énergie lorsque la dose d'énergie distribuée est supérieure à la dose d'énergie cible minimale et la puissance retournée est supérieure à une puissance retournée souhaitée.

2. Système selon la revendication 1, où l'interface utilisateur est configurée pour afficher une ou plusieurs données de patient associées à l'augmentation ou à la réduction de l'au moins une dose d'énergie cible.

3. Système selon la revendication 2, dans lequel la ou les données de patient comprennent des données sélectionnées à partir du groupe constitué d'une caractéristique anatomique, d'une mesure anatomique, d'une procédure médicale précédente, d'une condition médicale, ou d'une tolérance à la douleur inférieure.

4. Système selon la revendication 1, où l'interface utilisateur est configurée pour afficher une alerte lorsque la dose d'énergie appliquée est supérieure à la dose d'énergie cible minimale.

5. Système selon la revendication 1, où l'interface utilisateur est configurée pour afficher une ou plusieurs pertes pendant la distribution d'énergie.

6. Système selon la revendication 1, où l'interface utilisateur est configurée pour afficher un ou plusieurs paramètres d'énergie pendant la distribution d'énergie.

7. Système selon la revendication 6, dans lequel le paramètre d'énergie est la dose d'énergie totale distribuée à un tissu, et est calculé à partir de la valeur d'une puissance retournée.

8. Système selon la revendication 1, dans lequel la perte pendant la distribution d'énergie comprend une ou plusieurs pertes sélectionnées parmi le groupe composé de : un rayonnement, un chauffage diélectrique, une conduction, une convection, une réflexion, et une génération de buée ou de vapeur.

9. Système selon la revendication 1, où le signal est configuré pour modifier l'application de la dose d'énergie appliquée.

10. Système selon la revendication 1, dans lequel l'unité de mémoire est en outre configurée pour stocker au moins un paramètre de distribution d'énergie.

11. Système selon la revendication 1, où la source d'énergie est configurée pour amener le dispositif de distribution d'énergie à produire un effet thermique.

12. Système selon la revendication 1, comprenant en outre des moyens pour permettre à un utilisateur de modifier un paramètre de distribution d'énergie pendant l'utilisation.
